# EUROPEAN PATENT APPLICATION

(11) **EP 4 218 660 A1**
(43) Date of publication of application: **02.08.2023**
(21) Application number: 22216926.0
(22) Date of filing: 28.12.2022
(51) Int. Cl.: A61C 7/00, G16H 50/70, G16H 20/40, G16H 30/40, G16H 50/50

(54) **METHOD AND DEVICE FOR GENERATING ORTHODONTIC THEETH ALIGNMENT SHAPE**

(30) Priority: 27.01.2022 KR 20220012266
(71) Applicant: DDH INC., Seoul 03080 (KR)
(72) Inventor: Her, Soobok, 06608 Seoul (KR); Lee, Shin Jae, 10372 Goyang-si Gyeonggi-do (KR); Cho, Sungjoo, 06608 Seoul (KR)
(74) Representative: Gulde & Partner

(57) **Abstract**

The present invention relates to a method for generating an orthodontic teeth alignment shape for an orthodontic treatment, the method including the steps of: extracting specific points from a plurality of teeth in at least one of the upper and lower jaws, based on a patient's teeth shape information; generating a three-dimensional patient arch form based on the extracted specific points; comparing the patient arch form with a plurality of standard arch forms to select the standard arch form most similar to the patient arch form from the plurality of standard arch forms; and generating the orthodontic teeth alignment shape based on the selected standard arch form.

## Description

### BACKGROUND OF THE INVENTION

### Cross Reference to Related Application of the Invention

The present application claims the benefit of Korean Patent Application No. 10-2022-0012266 filed in the Korean Intellectual Property Office on January 27, 2022, the entire contents of which are incorporated herein by reference.

### Field of the Invention

The present invention relates to a method and device for generating an orthodontic teeth alignment shape for an orthodontic treatment.

### Background of the Related Art

An orthodontic treatment for correcting teeth alignment is generally conducted for a medical or beauty purpose. The orthodontic treatment is performed to correct various skeleton disharmony that may be generated in a patient's growing and living processes as well as to correct his or her misaligned teeth in position, so that the functions of normal teeth may be exerted well to provide a healthy oral structure. The skeleton disharmony has an influence on the outer appearance of the patient as well as his or her facial skeleton structure, which is one of important treatment subjects.

So as to allow the teeth to be fixed in right position or at normal angles during the orthodontic treatment, generally, a treatment in which a given force is applied to a tooth to move the tooth to a desired position within the alveolar bone is generally carried out. The orthodontic treatment may be performed through various methods, and for example, an orthodontic appliance using brackets and a wire made of a metal or ceramic material may be used. If the orthodontic appliance is used, the brackets are fixed to the surfaces of the teeth as the orthodontic treatment subjects, and an orthodontic wire is connected to the brackets, so that through the elastic or restoring force of the wire, an orthodontic force is applied to the teeth to gradually correct the misaligned teeth.

In the orthodontic treatment in which various orthodontic appliances are provided, therefore, there is a need to generate a standard teeth alignment shape as a target of a patient's orthodontic treatment and an orthodontic teeth alignment shape for making an orthodontic appliance based on the standard teeth alignment shape.

### SUMMARY OF THE INVENTION

Accordingly, the present invention has been made in view of the above-mentioned problems occurring in the related art, and it is an object of the present invention to provide a method and device for generating an orthodontic teeth alignment shape for an orthodontic treatment.

To accomplish the above-mentioned objects, according to an aspect of the present invention, there is provided a method for generating an orthodontic teeth alignment shape for an orthodontic treatment, the method including the steps of: extracting specific points from a plurality of teeth in at least one of the upper and lower jaws, based on a patient's teeth shape information; generating a three-dimensional patient arch form based on the extracted specific points; comparing the patient arch form with a plurality of standard arch forms to select the standard arch form most similar to the patient arch form from the plurality of standard arch forms; and generating the orthodontic teeth alignment shape based on the selected standard arch form.

According to the present invention, desirably, the specific points are Facial Axis points (FA points) of the respective teeth.

According to the present invention, desirably, the method may further include the step of acquiring the plurality of standard arch forms from teeth shape information of a plurality of normal occlusion persons.

According to the present invention, desirably, the step of acquiring the plurality of standard arch forms may include the steps of: extracting specific points from a plurality of teeth in at least one of the upper and lower jaws, based on the teeth shape information of the plurality of normal occlusion persons; performing three-dimensional cluster analysis for the extracted specific points from the teeth shape information of the plurality of normal occlusion persons; and generating the plurality of standard arch forms three-dimensionally, based on the results of the three-dimensional cluster analysis.

According to the present invention, desirably, in the step of generating the plurality of standard arch forms, the curve of occlusion may be reflected on the cluster analysis results to generate the plurality of standard arch forms.

According to the present invention, desirably, the step of selecting the standard arch form most similar to the patient arch form may include the steps of: generating a three-dimensional reference plane defined by the extracted specific points and having the patient arch form; projecting the plurality of standard arch forms on the reference plane; and comparing the patient arch form with the plurality of standard arch forms projected on the reference plane.

According to the present invention, desirably, in the step of generating a three-dimensional reference plane, the reference plane may be generated based on a plurality of planes defined by connecting a given reference point in a three-dimensional space and the neighboring two specific points of the extracted specific points to one another.

According to the present invention, desirably, in the step of comparing the patient arch form with the plurality of standard arch forms, the most similar standard arch form to the patient arch form may be selected based on the areas of spaces generated by overlaying the plurality of standard arch forms projected on the reference plane and the patient arch form on one another.

To accomplish the above-mentioned objects, according to another aspect of the present invention, there is provided a device for generating an orthodontic teeth alignment shape for an orthodontic treatment, including: a processor; and a memory for storing a program executable by the processor, wherein the processor extracts specific points from a plurality of teeth in at least one of the upper and lower jaws, based on a patient's teeth shape information, generates a three-dimensional patient arch form based on the extracted specific points, compares the patient arch form with a plurality of standard arch forms to select the standard arch form most similar to the patient arch form from the plurality of standard arch forms, and generates the orthodontic teeth alignment shape based on the selected standard arch form.

### BRIEF DESCRIPTION OF THE DRAWINGS

The above and other objects, features and advantages of the present invention will be apparent from the following detailed description of the embodiments of the invention in conjunction with the accompanying drawings, in which:
FIG. 1 is a block diagram showing an orthodontic teeth alignment shape generating system according to the present invention;
FIG. 2 is a flowchart showing a standard arch form generating method according to the present invention;
FIG. 3 is a flowchart showing an orthodontic teeth alignment shape generating method according to the present invention;
FIG. 4 is a flowchart showing step S340 of FIG. 3;
FIGs. 5 and 6 are perspective views showing the step of setting facial axis points on a plurality of teeth according to the present invention;
FIGs. 7 to 9 are exemplary views showing the standard arch forms generated by the method of FIG. 2;
FIG. 10 is a graph showing the step of generating a patient arch form according to the present invention;
FIGs. 11 and 12 are graphs showing the step of comparing the patient arch form with the standard arch forms according to the present invention; and
FIG. 13 is a block diagram showing an orthodontic teeth alignment shape generating device according to the present invention.

### DETAILED DESCRIPTION OF THE EMBODIMENTS

The present invention may be modified in various ways and may have several exemplary embodiments. Specific exemplary embodiments of the present invention are illustrated in the drawings and described in detail in the detailed description. However, this does not limit the invention within specific embodiments and it should be understood that the invention covers all the modifications, equivalents, and replacements within the idea and technical scope of the invention.

If it is determined that the detailed explanation on the well known technology related to the present invention makes the scope of the present invention not clear, the explanation will be avoided for the brevity of the description. Terms, such as the first, the second, and the like may be used to describe various elements, but the elements should not be restricted by the terms. The terms are used to only distinguish one element from the other element.

When it is said that one element is described as being "connected" or "coupled" to the other element, one element may be directly connected or coupled to the other element, but it should be understood that another element may be present between the two elements.

The terms "unit", "-or/er" and "module" described in the specification indicate a unit for processing at least one function or operation, which may be implemented by hardware, software or a combination thereof, such as a processor, a microprocessor, a microcontroller, a Central Processing Unit (CPU), a Graphics Processing unit (GPU), an Accelerate Processor Unit (APU), a Digital Signal Processor (DSP), an Application Specific Integrated Circuits (ASIC), a Field Programmable Gate Array (FPGA), and the like.

Moreover, the parts as will be discussed in the specification are just divided according to their main functions. That is, two or more parts as will be discussed below may be added as one part or one part may be divided into two or more parts according to more specified functions. The respective parts as will be discussed below may additionally perform some or all of functions performed by other parts as well as their main functions, and of course, also, some of the main functions of the respective parts may be performed only by other parts.

Hereinafter, embodiments of the present invention will be described in detail with reference to the attached drawings.

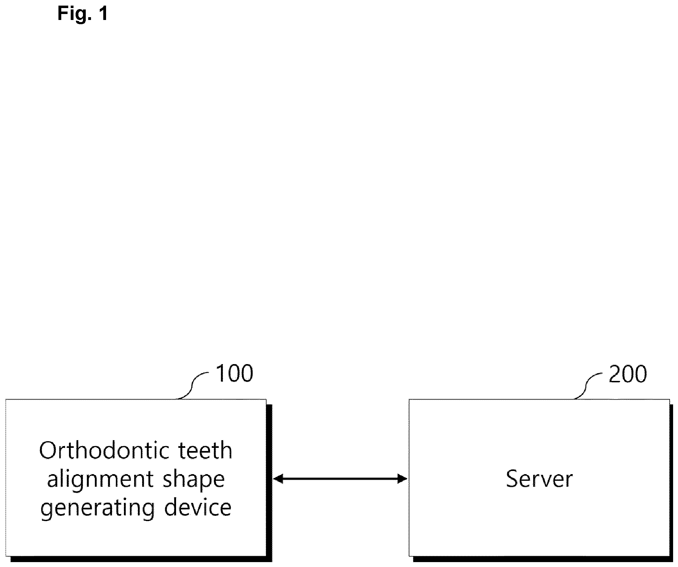

FIG. 1 is a block diagram showing an orthodontic teeth alignment shape generating system according to the present invention.

Referring to FIG. 1, an orthodontic teeth alignment shape generating system according to the present invention includes an orthodontic teeth alignment shape generating device 100 and a server 200.

The orthodontic teeth alignment shape generating device 100 acquires a patient's teeth shape information. To perform an orthodontic treatment, it is necessary to measure the patient's oral cavity. The patient's teeth shape information includes the shapes, positions, and directions of a plurality of teeth located in the oral cavity. The teeth shape information may include the subgingival area below the gingival line. The orthodontic teeth alignment shape generating device 100 may be configured to have a teeth shape information acquiring unit adapted to directly acquire the patient's teeth shape information or may be configured to receive the patient's teeth shape information pre-generated from the outside.

According to the present invention, the patient's teeth shape information may be acquired by means of scanning. The scanning is performed by using a teeth shape information acquiring device such as an optical 3D scanner, treatment CT, and the like, to acquire the patient's oral cavity information and to then obtain the entire teeth shape information by processing the acquired oral cavity information. The scanning method using the optical 3D scanner or CT is useful because the teeth shape information can be acquired, without having any physical contact with the patient's teeth or gum.

According to the present invention, the patient's teeth shape information may be acquired by means of plaster casting. The plaster casting is performed by applying a plaster material curable to the patient's oral cavity, making impressions of the teeth in the patient's oral cavity through the cured teeth shapes, and acquiring the teeth shape information. However, the plaster casting may make the patient feel uncomfortable and require longer time than the scanning in acquiring the teeth shape information.

The teeth shape information acquired by means of the scanning or plaster casting is converted into a three-dimensional (3D) model through digital processing.

According to the present invention, the teeth shape information may include both of the shapes of the plurality of teeth and the shape of the gum. The orthodontic teeth alignment shape generating device 100 distinguishes the plurality of teeth and the gum included in the teeth shape information from each other. For example, the orthodontic teeth alignment shape generating device 100 distinguishes the plurality of teeth from the gum, based on information such as color tone and brightness differences therebetween, pre-set boundaries therebetween, and the like. However, the information used for the distinguishing operation of the orthodontic teeth alignment shape generating device 100 may not be limited to the above-mentioned examples.

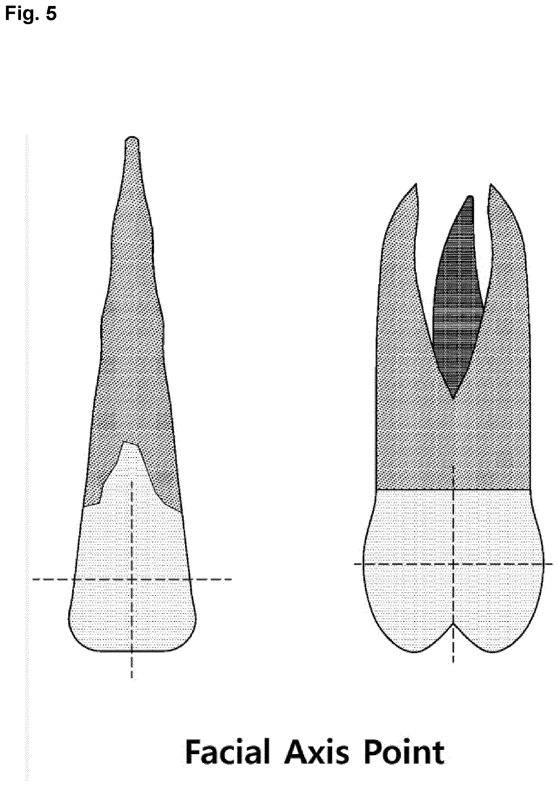

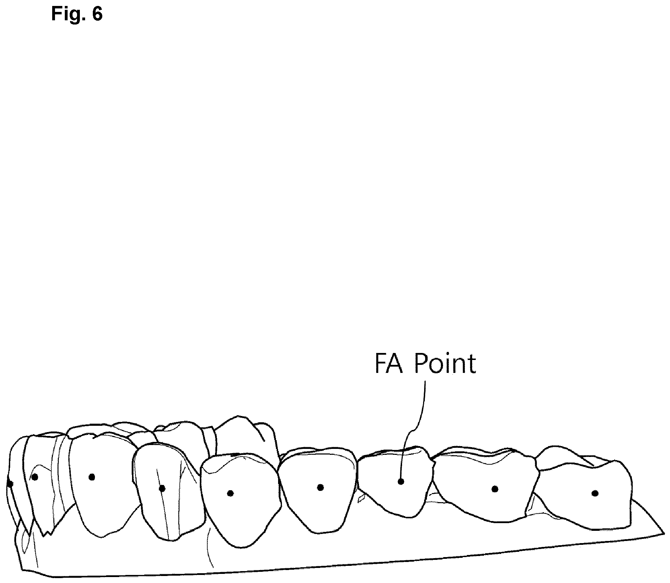

The orthodontic teeth alignment shape generating device 100 extracts specific points from a plurality of teeth in at least one of the patient's upper and lower jaws, based on the acquired teeth shape information. In this case, the specific points may be the Facial Axis points (FA points) of the respective teeth, and a detailed explanation of the facial axis points will be discussed later with reference to FIGs. 5 and 6.

The orthodontic teeth alignment shape generating device 100 generates a patient arch form and a three-dimensional reference plane including the patient arch form, based on the extracted specific points, and projects a plurality of standard arch forms stored in a database on the reference plane to compare the patient arch form and the standard arch forms.

The orthodontic teeth alignment shape generating device 100 selects the standard arch form that is most similar to the patient arch form from the plurality of standard arch forms, based on the comparison result. In this case, the plurality of standard arch forms may be obtained by performing statistical 3D cluster analysis for the specific points (that is, the facial axis points) extracted from the teeth shape information of a plurality of normal occlusion persons.

The orthodontic teeth alignment shape generating device 100 generates an orthodontic teeth alignment shape based on the selected standard arch form. The orthodontic teeth alignment shape is the shape for modeling an orthodontic appliance for converting the patient's teeth into the teeth alignment (that is, the standard teeth alignment shape) corresponding to the selected standard arch form. According to the present invention, the orthodontic teeth alignment shape may be provided to convert the patient's teeth into the standard teeth alignment shape at once, but so as to reduce the load applied to the patient, the orthodontic teeth alignment shape may be provided a plurality of times to gradually convert the patient's teeth into the standard teeth alignment shape so that the entire conversion target becomes the standard teeth alignment shape.

The server 200 communicates with the orthodontic teeth alignment shape generating device 100, while providing the information necessary for the operations for generating the orthodontic teeth alignment shape to the orthodontic teeth alignment shape generating device 100. Further, the server 200 stores an orthodontic appliance modeling provided from the orthodontic teeth alignment shape generating device 100 and provides the modeling to an orthodontic appliance generating device to apply the modeling to a real orthodontic appliance.

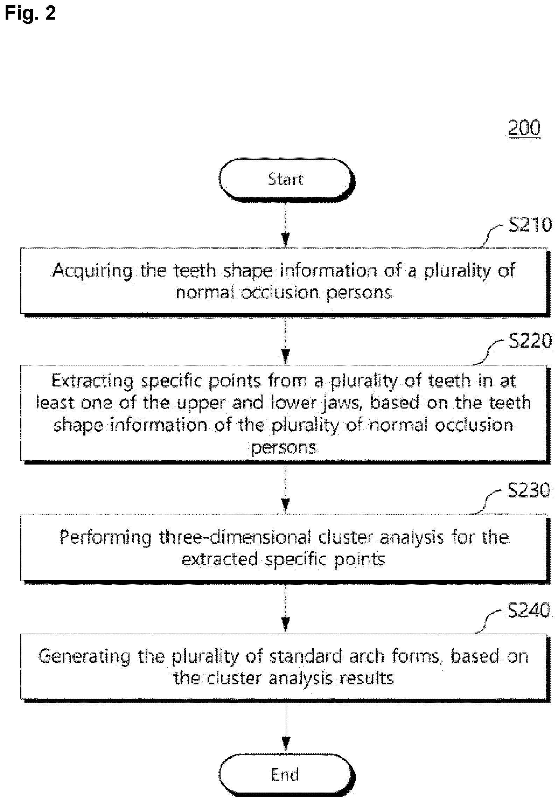

FIG. 2 is a flowchart showing a standard arch form generating method according to the present invention.

At step S210, the orthodontic teeth alignment shape generating device 100 acquires the teeth shape information of the plurality of normal occlusion persons.

For example, the orthodontic teeth alignment shape generating device 100 receives the teeth shape information of a plurality of subjects sorted as normal occlusion persons by dental doctors from an internal database and/or an external database server and thus acquires the teeth shape information of the plurality of normal occlusion persons.

In this case, the teeth shape information is generated by acquiring the oral cavity information using the information acquiring device such as an optical 3D scanner, treatment CT, and the like, and processing the acquired oral cavity information.

At step S220, the orthodontic teeth alignment shape generating device 100 extracts specific points from a plurality of teeth in the upper and/or lower jaws, based on the acquired teeth shape information of the respective normal occlusion persons. In this case, the specific points may be the Facial Axis points (FA points) of the respective teeth, and the extracted specific points are represented as specific coordinates in a 3D space.

According to the present invention, the orthodontic teeth alignment shape generating device 100 extracts the facial axis points based on a user's input. For example, the orthodontic teeth alignment shape generating device 100 provides a user interface to a user terminal connected thereto through communication or to a display part disposed therein, and thus extracts the facial axis points by selecting one point of the respective teeth based on the teeth shape information displayed on the user interface.

According to the present invention, the orthodontic teeth alignment shape generating device 100 extracts the facial axis points from the teeth shape information through a learned network function.

The network function is made by pre-learning the detection of the facial axial points of the respective teeth through learning data (for example, teeth shape information having labeled facial axial points of teeth by dental doctors or other network functions).

At step S230, the orthodontic teeth alignment shape generating device 100 performs statistical 3D cluster analysis for the extracted specific points (facial axis points) from the plurality of normal occlusion persons.

Through the cluster analysis, the specific points extracted from the respective teeth build a plurality of groups at specific positions in the 3D space.

At step S240, the orthodontic teeth alignment shape generating device 100 generates a plurality of standard arch forms based on the results of the 3D cluster analysis.

Each standard arch form is a curve defined in the 3D space and formed by connecting a plurality of groups sorted through the cluster analysis to one another in a given method. In this case, the curve of occlusion is reflected in generating the standard arch form. The curve of occlusion is defined as occlusal curvatures (the curve of Spee, the curve of Wilson, or the like) seen in the sagittal plane and frontal plane, and accordingly, the orthodontic teeth alignment shape generating device 100 reflects the curve of occlusion to generate the plurality of standard arch forms in the 3D space.

According to the present invention, further, the standard arch forms having different numbers and shapes may be set based on the races, sexes, age distributions, and the like of the normal occlusion persons.

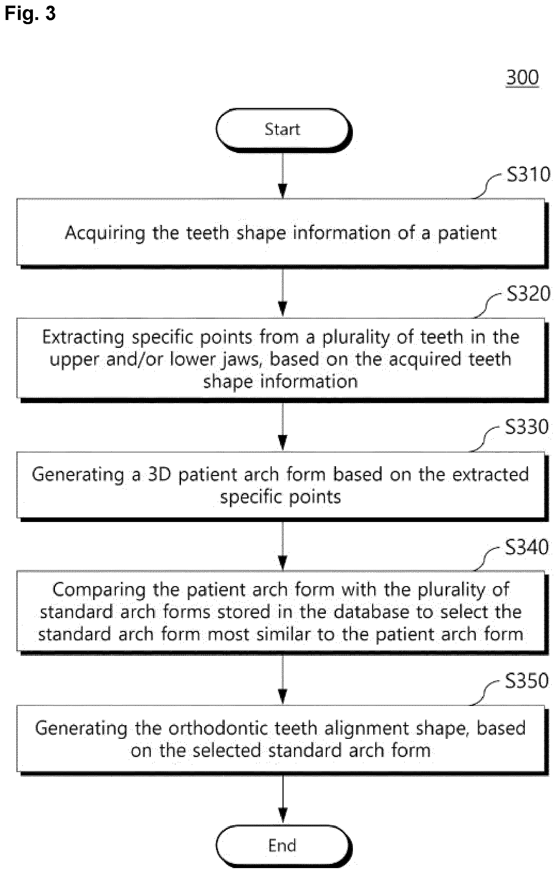

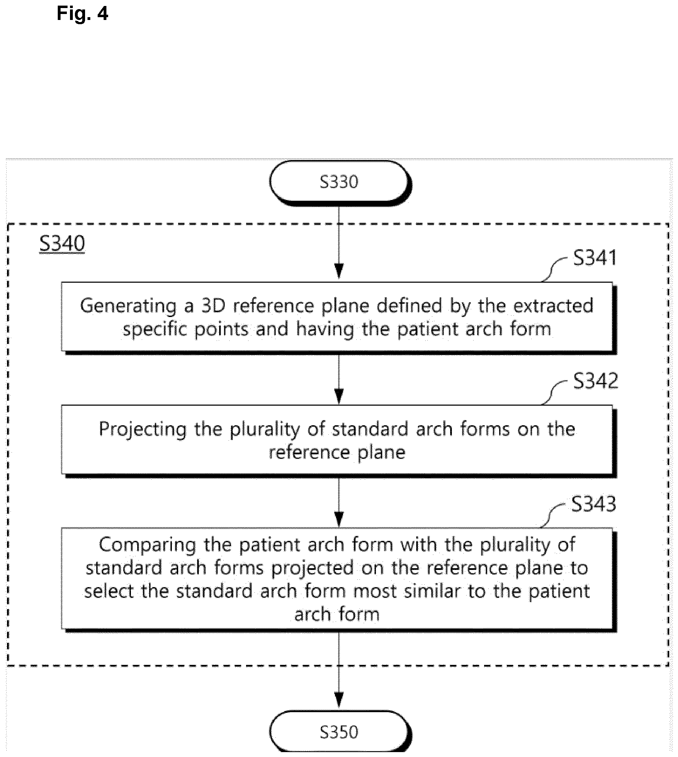

FIG. 3 is a flowchart showing an orthodontic teeth alignment shape generating method according to the present invention, and FIG. 4 is a flowchart showing the step S340 of FIG. 3.

At step S310, the orthodontic teeth alignment shape generating device 100 acquires the teeth shape information of a patient (that is, a subject for an orthodontic treatment).

For example, the orthodontic teeth alignment shape generating device 100 receives the teeth shape information of the patient from an external computing device (server) connected thereto through wired and/or wireless communication or though an information acquiring device such as an optical 3D scanner, treatment CT, and the like.

At step S320, the orthodontic teeth alignment shape generating device 100 extracts specific points from a plurality of teeth in the upper and/or lower jaws, based on the acquired teeth shape information of the patient. In this case, the specific points may be the Facial Axis points (FA points) of the respective teeth, and the extracted specific points are represented as specific coordinates in a 3D space.

According to the present invention, the orthodontic teeth alignment shape generating device 100 extracts (generates) the facial axis points based on a user's input or a learned network function.

At step S330, the orthodontic teeth alignment shape generating device 100 generates a 3D patient arch form based on the extracted specific points. For example, the orthodontic teeth alignment shape generating device 100 connects the plurality of specific points represented as the 3D coordinates to one another and thus generates the 3D patient arch form.

At step S340, the orthodontic teeth alignment shape generating device 100 compares the patient arch form with the plurality of standard arch forms stored in a database to select the standard arch form most similar to the patient arch form from the plurality of standard arch forms. In this case, the standard arch forms are generated based on the teeth shape information of the normal occlusion persons through the method 200 explained with reference to FIG. 2 and then stored in the database. Each standard arch form represents a 3D curve.

According to the present invention, the step S340 includes steps S341 to S343 as shown in FIG. 4.

At step S341, first, the orthodontic teeth alignment shape generating device 100 generates a 3D reference plane defined by the extracted specific points and having the patient arch form.

In specific, the orthodontic teeth alignment shape generating device 100 generates the reference plane based on a plurality of planes defined by connecting a given reference point in the 3D space and the neighboring two specific points of the extracted specific points to one another. According to the present invention, the reference point is a starting point in the 3D space to which the patient arch form is mapped.

That is, one plane is defined by the two straight lines passing through the neighboring two specific points from the given reference point, and accordingly, the plurality of planes are defined by connecting the given reference point and the neighboring two specific points to one another. As a result, the planes are coupled to one another to generate the 3D reference plane. In this case, the patient arch form is disposed on the reference plane.

At step S342, next, the orthodontic teeth alignment shape generating device 100 projects the plurality of standard arch forms on the reference plane.

For example, the orthodontic teeth alignment shape generating device 100 aligns the plurality of standard arch forms with the patient arch form, based on the reference point, and projects the plurality of standard arch forms on the reference plane.

At step S343, the orthodontic teeth alignment shape generating device 100 compares the patient arch form with the plurality of standard arch forms projected on the reference plane to select the standard arch form most similar to the patient arch form from the plurality of standard arch forms.

According to the present invention, the orthodontic teeth alignment shape generating device 100 selects the standard arch form most similar to the patient arch form, based on a minimal value of an area or distance of a space made by overlaying the plurality of standard arch forms and the patient arch form projected on the reference plane on one another.

After that, at step S350, the orthodontic teeth alignment shape generating device 100 generates the orthodontic teeth alignment shape to be applied to the patient, based on the selected standard arch form.

In this case, the orthodontic teeth alignment shape is the shape for modeling an orthodontic appliance for converting the patient's teeth into the teeth alignment (that is, the standard teeth alignment shape) corresponding to the selected standard arch form.

FIGs. 5 and 6 are perspective views showing the step of setting facial axis points on a plurality of teeth according to the present invention.

FIG. 5 shows an example of a tooth having a crown and a root. Referring to FIG. 5, the facial axis point of the tooth is the bisector point of the clinical crown separated from the gingival in occlusion, that is, the crossing point of the axes shown.

Referring to FIG. 6, the facial axis points of the respective teeth are represented to the forms of points. The facial axis points of the respective teeth are extracted independently of one another and mapped as coordinates in the 3D space. The line connecting the facial axis points of the respective teeth represents the arch form.

According to the present invention, the orthodontic teeth alignment shape generating device 100 distinguishes the long axes of the teeth based on the respective teeth shapes and determines the facial axis points of the respective teeth according to the long axes, without having any input of the user.

According to the present invention, the orthodontic teeth alignment shape generating device 100 extracts the facial axis points of the respective teeth from the teeth shape information through a learned network function.

The network function has the same meaning as a neural network. In this case, the neural network is composed of a group of calculation units connected to one another, which are called nodes, and the nodes are called neurons. Generally, the neural network is composed of a plurality of nodes. The nodes constituting the neural network are connected to one another by means of one or more links.

Some of the nodes constituting the neural network build one layer according to distances from an initial input node. For example, the nodes having the distance of n from the initial input node build an n layer. The neural network may include a Deep Neural Network (DNN) with a plurality of hidden layers as well as input and output layers.

The network function may include a plurality of convolutional layers and a plurality of fully connected layers. The plurality of convolutional layers serve to extract the features of an image by means of the abstraction of the image, and the plurality of fully connected layers serve to estimate the output probability of a detected object.

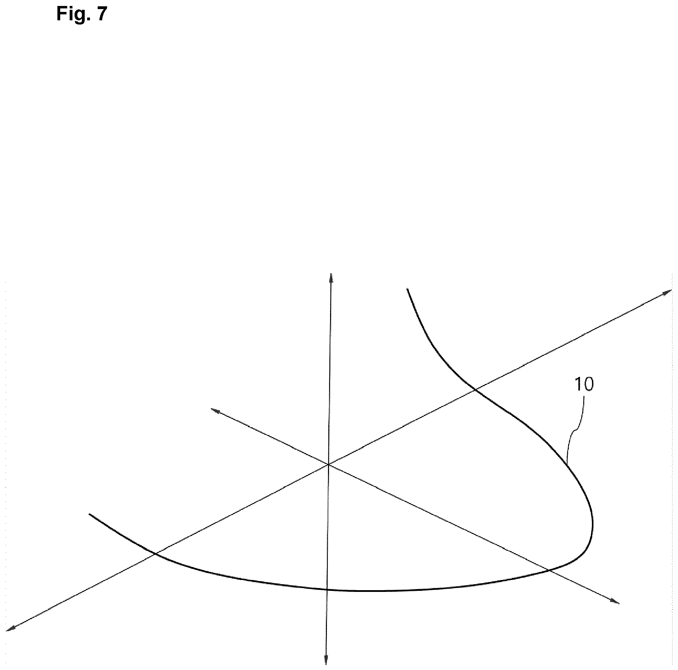

FIGs. 7 to 9 are exemplary views showing the standard arch forms generated by the method of FIG. 2.

The standard arch form 10 is obtained by performing the 3D cluster analysis for the specific points (that is, the facial axis points) extracted from the respective teeth, based on the teeth shape information of the plurality of normal occlusion persons.

As shown in FIG. 7, the standard arch form 10 has a given curve in the 3D space. In this case, the curvature of the standard arch form 10 is determined by reflecting the curve of occlusion.

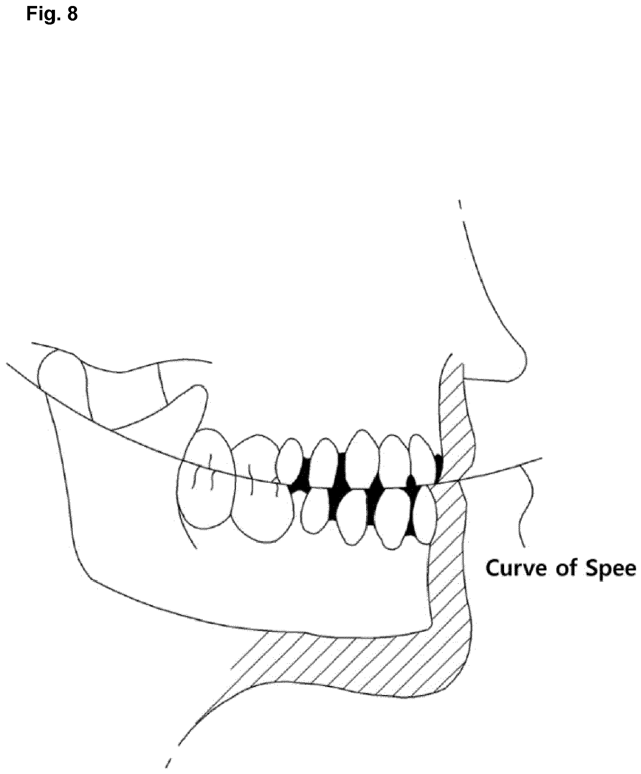

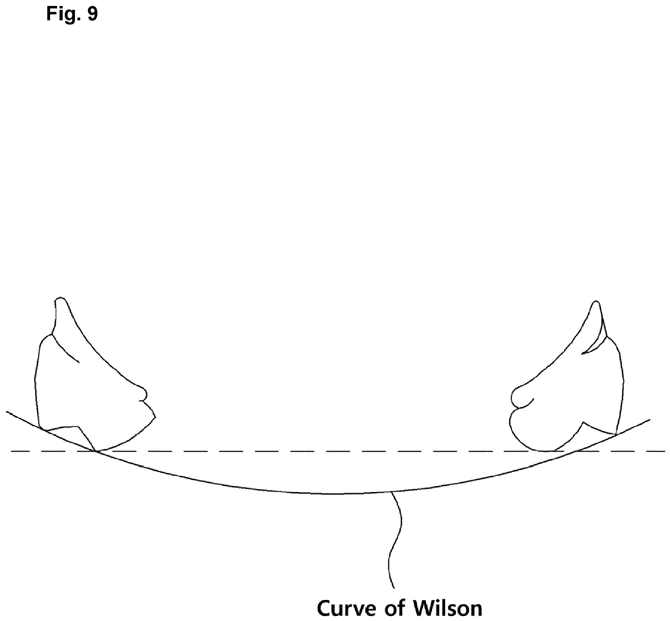

Referring to FIGs. 8 and 9, the curve of occlusion represents an occlusal curvature (the curve of Spee) seen in the sagittal plane and an occlusal curvature (the curve of Wilson) seen in the frontal plane.

In FIG. 7, one standard arch form is shown, but it is just exemplary. Three or more standard arch forms may be obtained according to the cluster analysis results.

According to the present invention, further, the standard arch forms having different numbers and shapes may be set based on the races, sexes, age distributions, and the like of the normal occlusion persons.

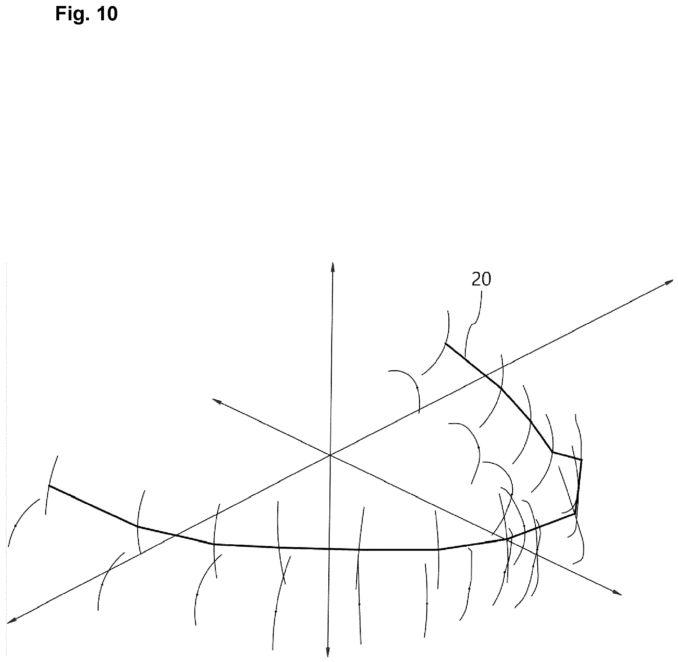

FIG. 10 is a graph showing the step of generating the patient arch form according to the present invention.

The facial axis points determined for the plurality of teeth are represented as coordinates in the 3D space, and as shown in FIG. 10, the 3D coordinates of the facial axis points are calculated around a given reference point. The reference point is desirably set so that it exists inside the patient arch form generated by connecting the facial axis points to one another.

The 3D coordinates of the respective facial axis points are calculated by extending x-, y- and z-axes from the reference point. In this case, the 3D coordinates of the reference point is 0, 0, 0. FIG. 10 shows the 3D coordinates of the facial axis points of the upper jaw, for the conveniences of the description.

The facial axis points in the 3D space are connected to one another to generate the 3D patient arch form 20.

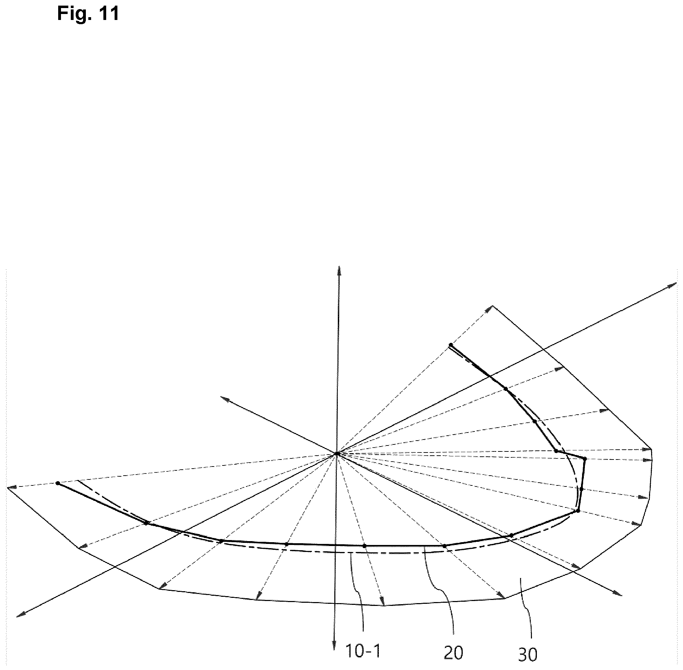

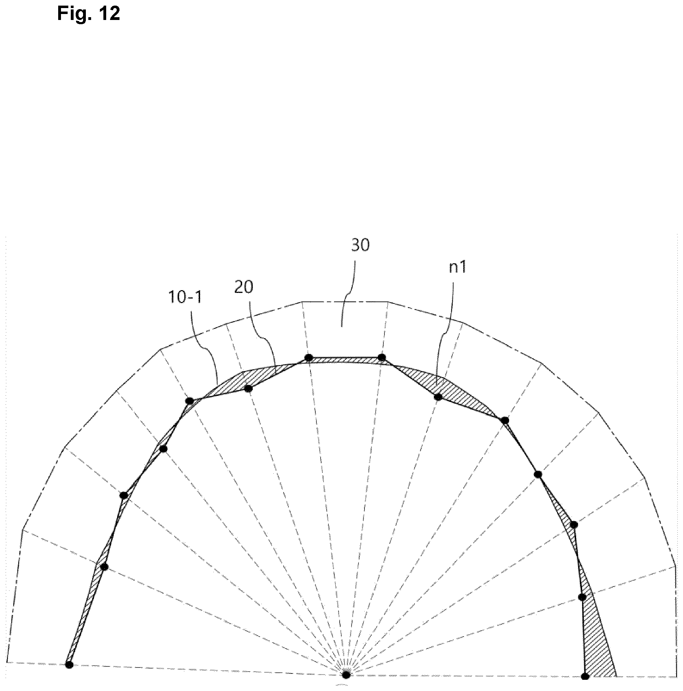

FIGs. 11 and 12 are graphs showing the step of comparing the patient arch form with the standard arch forms according to the present invention.

According to the present invention, the 3D reference plane 30 is generated to compare the patient arch form 20 with the plurality of standard arch forms 10.

The 3D reference plane 30 is generated by selecting the neighboring two facial axis points sequentially and coupling the planes defined by the two straight lines passing through the two facial axis points from the reference point to one another.

The patient arch form 20 generated by connecting the facial axis points to one another is disposed on the reference plane 30.

Next, the plurality of standard arch forms 10 are projected on the reference plane 30 simultaneously or sequentially, and the standard arch form 10-1 projected on the reference plane 30 is compared with the patient arch form 20 to select the standard arch form 10 most similar to the patient arch form 20.

As shown in FIG. 12, the most similar standard arch form 10 to the patient arch form 20 is selected based on the areas of spaces n1 generated by overlaying the standard arch form 10-1 and the patient arch form 20 on each other.

For example, the plurality of standard arch forms 10 are projected on the reference plane 30 sequentially to calculate the areas of the spaces n1, and the standard arch form 10 having the minimal areas of the spaces n1 is selected as the most similar standard arch form 10 to the patient arch form 20.

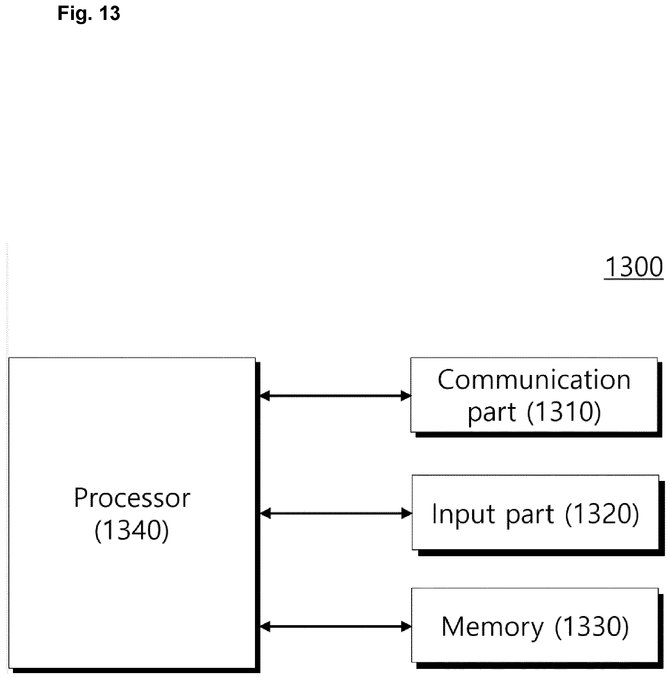

FIG. 13 is a block diagram showing the orthodontic teeth alignment shape generating device 1300 according to the present invention.

A communication part 1310 transmits and receives data (for example, the teeth shape information, and the like) to and from an external device and/or an external server. The communication part 1310 may include a wired and/or wireless communication part. If the communication part 1310 includes the wired communication part, it may include one or more networks of a Local Area Network (LAN), a Wide Area Network (WAN), a Value Added Network (VAN), a mobile radio communication network, a satellite communication network, and a combination thereof, which enable communication. If the communication part 1310 includes the wireless communication part, it may transmit and receive data or signals wirelessly using cellular communication, a wireless LAN (e.g., Wi-Fi), and the like. According to the present invention, the communication part 1310 transmits and receives the data or signals to and from the external device and/or external server under the control of a processor 1340 as will be discussed later.

An input part 1320 receives various user inputs and/or commands through external control. To do this, the input part 1320 includes one or more input devices or connects one or more input devices to one another. For example, the input part 1320 is connected to an interface for various inputs such as a keypad, a mouth, and the like and thus receives the user inputs and/or commands. To do this, the input part 1320 may include interfaces such as a USB port, Thunderbolt, and the like. Further, the input part 1320 may include various input devices such as a touch screen, buttons, and the like or may be connected thereto to receive the user inputs and/or commands from the outside.

The memory 1330 stores the program for operating the processor 1340 and temporarily or permanently stores the data inputted to or outputted from the orthodontic teeth alignment shape generating device 1300. Further, the memory 1330 may include at least one of a flash memory, a hard disk memory, a multimedia card micro type memory, a card type memory (for example, SD or XD memory), a Random Access Memory (RAM), a Static Random Access Memory (SRAM), a Read-Only Memory (ROM), an Electrically Erasable Programmable Read-Only Memory (EEPROM), a Programmable Read-Only Memory (PROM), a magnetic memory, a magnetic disk, and an optical disk.

The memory 1330 stores various network functions and algorithms and further stores various data, programs (with one or more instructions), applications, software, commands, codes, and the like for operating and controlling the orthodontic teeth alignment shape generating device 1300.

The processor 1340 controls the whole operation of the orthodontic teeth alignment shape generating device 1300. The processor 1340 executes one or more programs stored in the memory 1330. The processor 1340 represents a Central Processing Unit (CPU), a Graphics Processing Unit (GPU), or a dedicated processor with which the methods of the present invention are performed.

According to the present invention, the processor 1340 extracts the specific points of respective teeth in at least one of a patient's upper and lower jaws, based on the patient's teeth shape information, generates a 3D patient arch form based on the extracted specific points, compares the patient arch form with a plurality of standard arch forms, selects the standard arch form most similar to the patient arch form from the plurality of standard arch forms, and generates the orthodontic teeth alignment shape based on the selected standard teeth alignment form.

In this case, the specific points are Facial Axis points (FA points) of the respective teeth.

According to the present invention, the processor 1340 acquires the plurality of standard arch forms from the teeth shape information of a plurality of normal occlusion persons.

According to the present invention, the processor 1340 extracts specific points from a plurality of teeth in at least one of the upper and lower jaws, based on the teeth shape information of the plurality of normal occlusion persons, performs statistical 3D cluster analysis for the extracted specific points from the teeth shape information of the plurality of normal occlusion persons, and generates the plurality of standard arch forms based on the results of the 3D cluster analysis.

According to the present invention, the processor 1340 reflects the curve of occlusion on the cluster analysis results to generate the plurality of standard arch forms.

According to the present invention, the processor 1340 generates a 3D reference plane defined by the extracted specific points and having the patient arch form, projects the plurality of standard arch forms on the reference plane, and compares the patient arch form with the plurality of standard arch forms projected on the reference plane.

According to the present invention, the processor 1340 generates the reference plane based on a plurality of planes defined by connecting a given reference point in the 3D space and the neighboring two specific points of the extracted specific points to one another.

According to the present invention, the processor 1340 selects the most similar standard arch form to the patient arch form based on the areas of spaces generated by overlaying the plurality of standard arch forms projected on the reference plane and the patient arch form on one another.

Even though not shown, further, the orthodontic teeth alignment shape generating device 1300 may further include a display part. The display part serves to display the information processed in the orthodontic teeth alignment shape generating device 1300. For example, the display part displays a user interface for providing the patient's teeth alignment shape information, the standard teeth alignment shape information as a target of the orthodontic treatment, or the orthodontic teeth alignment shape for making the orthodontic appliance, to the user. The display part may be used as an input device as well as the output device. The display part may include at least one of a liquid crystal display, a thin film transistor-liquid crystal display, an organic light-emitting diode, a flexible display, a 3D display, and an electrophoretic display.

The various embodiments of the present disclosure as described above may be implemented in the form of a program instruction that can be performed through various computers, and may be recorded in a computer readable recording medium. The computer readable medium may include a program command, a data file, a data structure, and the like independently or in combination. The program instruction recorded in the recording medium is specially designed and constructed for the present disclosure, but may be well known to and may be used by those skilled in the art of computer software. The computer readable recording medium may include a magnetic medium such as a hard disc, a floppy disc, and a magnetic tape, an optical recording medium such as a Compact Disc Read Only Memory (CD-ROM) and a Digital Versatile Disc (DVD), a magneto-optical medium such as a floptical disk, and a hardware device specifically configured to store and execute program instructions, such as a Read Only Memory (ROM), a Random Access Memory (RAM), and a flash memory. Further, the program command may include a machine language code generated by a compiler and a high-level language code executable by a computer through an interpreter and the like.

Further, the methods according to the embodiments of the present invention are included in a computer program product. The computer program product is used for trading between sellers and buyers.

The computer program product may include an S/W program and a computer readable recording medium in which the S/W program is stored. For example, the computer program product may include an S/W program product (e.g., a downloadable app) electronically distributed through a manufacturer of an electronic device or an electronic market (e.g., Google play store, an app store, and the like). To perform the electronic distribution, at least a portion of the S/W program is stored in a recording medium or temporarily generated. In this case, the recording medium may be a recording medium for a manufacturer server, a server of an electronic market, or a relay server in which the S/W program is temporarily stored.

The computer program product may include a recording medium of a server and a recording medium of a client device, in a system composed of the server and the client device. Otherwise, if a third device (e.g., a smartphone) communicating with the server or client device, the computer program product may include a recording medium of the third device. Otherwise, the computer program product may include an S/W program itself that is transmitted to the client device or the third device from the server or transmitted to the client device from the third device.

In this case, one of the server, the client device, and the third device executes the computer program product and performs the methods according to the embodiments of the present invention. Otherwise, two or more devices of the server, the client device, and the third device execute the computer program product and distributedly perform the methods according to the embodiments of the present invention.

For example, the server (e.g., a cloud server, an artificial intelligence server, and the like) executes the computer program product stored therein, and the client device communicating with the server performs the methods according to the embodiments of the present invention.

As described above, the method and device for generating the orthodontic teeth alignment shape for the orthodontic treatment according to the present invention can generate the orthodontic teeth alignment shape for correcting the patient's teeth alignment, in a fast, convenient, and accurate manner, and apply the generated orthodontic teeth alignment shape to the patient teeth alignment.

The present invention may be modified in various ways and may have several exemplary embodiments. Accordingly, it should be understood that the invention covers all the modifications, equivalents, and replacements within the idea and technical scope of the invention. Therefore, the present invention is not to be restricted by the embodiments as mentioned above. It is therefore intended that the scope of the invention be limited not by this detailed description, but rather by the claims appended hereto.

## Claims

1. A method for generating an orthodontic teeth alignment shape for an orthodontic treatment, the method comprising the steps of:
extracting specific points from a plurality of teeth in at least one of the upper and lower jaws, based on a patient's teeth shape information;
generating a three-dimensional patient arch form based on the extracted specific points;
comparing the patient arch form with a plurality of standard arch forms to select the standard arch form most similar to the patient arch form from the plurality of standard arch forms; and
generating the orthodontic teeth alignment shape based on the selected standard arch form.

2. The method according to claim 1, wherein the specific points are Facial Axis points (FA points) of the respective teeth.

3. The method according to claim 1, further comprising the step of acquiring the plurality of standard arch forms from teeth shape information of a plurality of normal occlusion persons.

4. The method according to claim 3, wherein the step of acquiring the plurality of standard arch forms comprises the steps of:
extracting specific points from a plurality of teeth in at least one of the upper and lower jaws, based on the teeth shape information of the plurality of normal occlusion persons;
performing three-dimensional cluster analysis for the extracted specific points from the teeth shape information of the plurality of normal occlusion persons; and
generating the plurality of standard arch forms three-dimensionally, based on the results of the three-dimensional cluster analysis.

5. The method according to claim 4, wherein in the step of generating the plurality of standard arch forms, the curve of occlusion is reflected on the cluster analysis results to generate the plurality of standard arch forms.

6. The method according to claim 1, wherein the step of selecting the standard arch form most similar to the patient arch form comprises the steps of:
generating a three-dimensional reference plane defined by the extracted specific points and having the patient arch form;
projecting the plurality of standard arch forms on the reference plane; and
comparing the patient arch form with the plurality of standard arch forms projected on the reference plane.

7. The method according to claim 6, wherein in the step of generating a three-dimensional reference plane, the reference plane is generated based on a plurality of planes defined by connecting a given reference point in a three-dimensional space and the neighboring two specific points of the extracted specific points to one another.

8. The method according to claim 6, wherein in the step of comparing the patient arch form with the plurality of standard arch forms, the most similar standard arch form to the patient arch form is selected based on the areas of spaces generated by overlaying the plurality of standard arch forms projected on the reference plane and the patient arch form on one another.

9. A device for generating an orthodontic teeth alignment shape for an orthodontic treatment, comprising:
a processor; and
a memory for storing a program executable by the processor,
wherein the processor extracts specific points from a plurality of teeth in at least one of the upper and lower jaws, based on a patient's teeth shape information, generates a three-dimensional patient arch form based on the extracted specific points, compares the patient arch form with a plurality of standard arch forms to select the standard arch form most similar to the patient arch form from the plurality of standard arch forms, and generates the orthodontic teeth alignment shape based on the selected standard arch form.

10. A computer program stored in a recording medium for executing the method according to any one of claims 1 to 9.
